(19) Europäisches Patentamt
European Patent Office
Office européen des brevets

(11) **EP 2 612 599 A1**

(12) **EUROPEAN PATENT APPLICATION**
published in accordance with Art. 153(4) EPC

(43) Date of publication:
**10.07.2013 Bulletin 2013/28**

(21) Application number: **11821435.2**

(22) Date of filing: **15.07.2011**

(51) Int Cl.:
**A61B 8/08** (2006.01)

(86) International application number:
**PCT/JP2011/066176**

(87) International publication number:
**WO 2012/029417 (08.03.2012 Gazette 2012/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2010 JP 2010194180**

(71) Applicant: **Hitachi Medical Corporation**
**Chiyoda-ku**
**Tokyo 101-0021 (JP)**

(72) Inventor: **WAKI,Koji**
**Tokyo 101-0021 (JP)**

(74) Representative: **MERH-IP**
**Matias Erny Reichl Hoffmann**
**Paul-Heyse-Strasse 29**
**80336 München (DE)**

(54) **ULTRASOUND DIAGNOSTIC DEVICE AND EVALUATION CALCULATION METHOD**

(57)    In order to improve the quality of a three-dimensional elasticity image by establishing a method of evaluating the quality of the elasticity volume data, there is provided an ultrasonic diagnostic apparatus including: a two-dimensional elasticity image forming unit 116 that calculates elasticity frame data indicating the elasticity distribution measured by scanning an object in a three-dimensional manner using an ultrasonic wave; an elasticity volume data generation unit 117 that generates elasticity volume data by collecting the plurality of elasticity frame data items; a three-dimensional elasticity image forming unit 118 that forms a three-dimensional elasticity image by performing volume rendering of the elasticity volume data; and a quality calculation unit 121 that calculates a volume evaluation value indicating the quality of the elasticity volume data on the basis of an autocorrelation value between a pair of tomographic frame data items, which are a basis for calculating the elasticity frame data, and a frame evaluation value indicating the quality of the elasticity frame data.

FIG.1

**Description**

Technical Field

The present invention relates to an ultrasonic diagnostic apparatus that displays a three-dimensional elasticity image, which shows the hardness or softness of body tissue of an object, using ultrasonic waves and an evaluation calculation method.

Background Art

The ultrasonic diagnostic apparatus transmits an ultrasonic wave to an object, receives an echo signal reflected from the body tissue inside the object, generates an ultrasonic image such as a three-dimensional elasticity image showing the hardness or softness of the body tissue, for example, and displays the ultrasonic image on a monitor to provide it for diagnosis. In addition, in order to contribute to the diagnosis by displaying the shape of the inside of the object intelligibly, a three-dimensional ultrasonic wave image of a three-dimensional tomographic image or a three-dimensional elasticity image is generated and displayed. On the other hand, in order to improve the diagnostic accuracy, improvements in the quality of an ultrasonic image, such as an improvement in the resolution of an image and noise reduction, are in demand.

For example, PTL 1 has proposed building a three-dimensional elasticity image by combining elasticity image sections of the same displacement or pressure when building a three-dimensional elasticity image by performing volume rendering of elasticity volume data. In addition, PTL 1 has also proposed building a three-dimensional elasticity image by combining elasticity images using an elasticity frame with a high correlation coefficient. That is, when building a three-dimensional elasticity image, elasticity volume data is configured by two-dimensional elasticity frame data items with the same amount of compression (displacement) among a plurality of two-dimensional elasticity frame data items acquired continuously, and a three-dimensional elasticity image is built by performing volume rendering of the elasticity volume data.

Citation List

Patent Literature

[PTL 1] JP-A-2008-259555

Summary of Invention

Technical Problem

Incidentally, although desired elasticity frame data is selected from elasticity volume data to combine the elasticity volume data in PTL 1, evaluating the quality of the entire elasticity volume data acquired is not taken into consideration. As a result, noise (for example, a streaky noise image) may appear in the three-dimensional elasticity image built by volume rendering.

It is an object of the present invention to improve the quality of a three-dimensional elasticity image by establishing a method of evaluating the quality of the elasticity volume data.

Solution to Problem

In order to solve the above-described problem, the present invention is an ultrasonic diagnostic apparatus including: an elasticity calculation unit that calculates elasticity frame data indicating the elasticity distribution measured by scanning an object in a three-dimensional manner using an ultrasonic wave; an elasticity volume generation unit that generates elasticity volume data by collecting the plurality of elasticity frame data items; a three-dimensional elasticity image forming unit that forms a three-dimensional elasticity image by performing volume rendering of the elasticity volume data; and a display unit that displays the three-dimensional elasticity image, and is characterized in that it includes a quality calculation unit that calculates a volume evaluation value indicating the quality of the elasticity volume data on the basis of a frame evaluation value indicating the quality of the elasticity frame data.

The volume evaluation value indicating the quality, which has been calculated as described above is displayed on the display unit so as to correspond to the three-dimensional elasticity image. In this manner, it is possible to determine how high the quality of the displayed three-dimensional elasticity image is. In addition, the volume evaluation value indicating the quality can be identified at a glance by displaying, on the display unit, marks, bar charts, or pie charts having different display forms corresponding to the volume evaluation value indicating the quality. This can contribute to improvement in diagnostic accuracy.

In addition, the present invention is not limited to the three-dimensional elasticity image, and may be configured to include a cross-sectional elasticity image forming section that displays three orthogonal cross-sectional elasticity images, which are formed on the basis of the elasticity volume data, or multiple cross-sectional elasticity images, which are obtained by slicing using a plurality of parallel cross-sections, on the display unit, or may be configured to include a display surface quality calculation section that calculates a volume evaluation value, which indicates the quality of each cross-sectional elasticity image, using the quality calculation unit and displays the volume evaluation value on the display unit.

As described above, according to the present invention, since the quality of a three-dimensional elasticity image can be displayed, the examiner can select a three-dimensional elasticity image easily. In addition, since it is also possible to reconstruct and display the high-quality three-dimensional elasticity image, high-level diagnostic support is possible.

Advantageous Effects of Invention

According to the present invention, since a method of evaluating the quality of the elasticity volume data can be established, it is possible to improve the quality of a three-dimensional elasticity image.

Brief Description of Drawings

Fig. 1 is a block diagram showing the overall configuration of an ultrasonic diagnostic apparatus of a first embodiment of the present invention.

Fig. 2 is a block diagram showing the configuration of a quality calculation unit of the first embodiment.

Fig. 3 is a diagram illustrating the processing procedure of a characteristic unit of the first embodiment.

Fig. 4 is a diagram illustrating the processing procedure of a characteristic unit of a second embodiment of the present invention.

Fig. 5 is a diagram illustrating the processing procedure of a characteristic unit of a third embodiment of the present invention.

Fig. 6 is a diagram illustrating the processing procedure in a modification of the third embodiment of the present invention.

Fig. 7 is a diagram illustrating a display example of a cross-sectional elasticity image of a fourth embodiment of the present invention.

Fig. 8 is a block diagram showing the configuration of main units to realize the display of the cross-sectional elasticity image of the fourth embodiment of the present invention.

Fig. 9 is a diagram showing an example of a method of displaying the volume evaluation value indicating the quality of elasticity volume data.

Fig. 10 is a diagram showing another example of the method of displaying the volume evaluation value indicating the quality of elasticity volume data.

Description of Embodiments

Embodiments will be described with reference to the drawings.

First embodiment

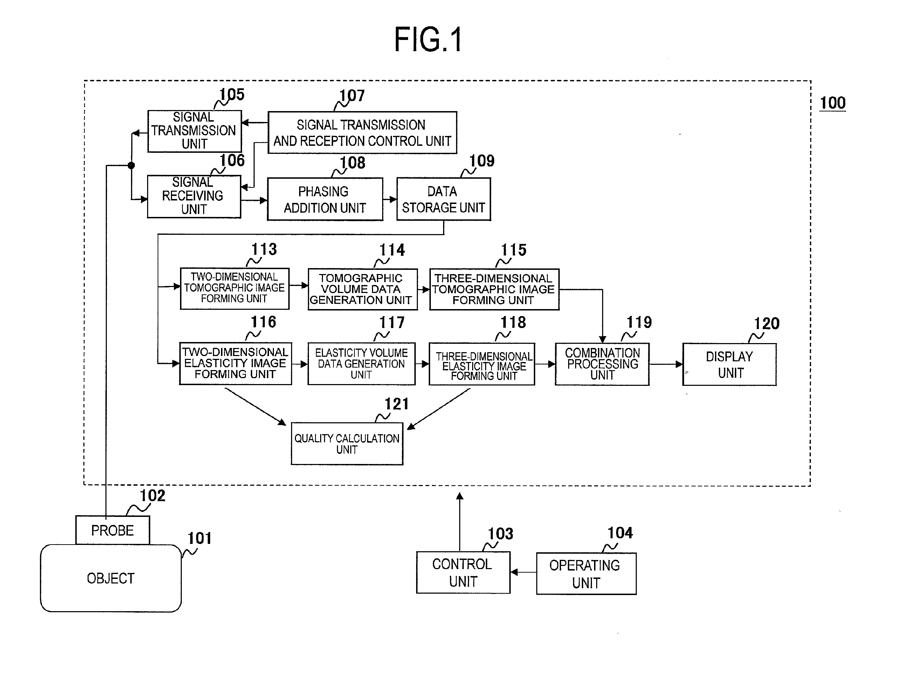

An ultrasonic diagnostic apparatus 100 of an embodiment to which the present invention is applied will be described using Fig. 1.

As shown in the drawing, the ultrasonic diagnostic apparatus 100 includes: an ultrasonic probe 102 used in contact with an object 101; a signal transmission unit 105 that transmits an ultrasonic wave repeatedly to the object 101 through the ultrasonic probe 102 at fixed intervals of time; a signal receiving unit 106 that receives a reflected echo signal from the inside of the object 101; a signal transmission and reception control unit 107 that controls the signal transmission unit 105 and the signal receiving unit 106; and a phasing addition unit 108 that performs phasing addition of the reflected echo received by the signal receiving unit 106.

The ultrasonic probe 102 has a function of transmitting and receiving an ultrasonic wave to and from the object 101 through a transducer. The ultrasonic probe 102 is formed by arraying a plurality of rectangular or fan-shaped transducers. The ultrasonic probe 102 is mechanically vibrated in a direction (short axis direction) perpendicular to the arrangement direction (longitudinal direction) of the plurality of transducers, so that a three-dimensional scan of the ultrasonic wave is possible. In addition, the three-dimensional scan of the ultrasonic wave is not limited to vibrating the ultrasonic probe 102 mechanically in the short axis direction. For example, electronic scanning in the short axis direction using an ultrasonic wave may be performed by using the ultrasonic probe 102 in which a plurality of transducers are arrayed in a two-dimensional manner.

The signal transmission unit 105 drives the transducers of the ultrasonic probe 102 to generate a carrier pulse for generating an ultrasonic wave. The signal transmission unit 105 has a function of setting the convergent point of the transmitted ultrasonic wave at the arbitrary depth. In addition, the signal receiving unit 106 generates an RF signal, that is, a received signal by amplifying the reflected echo signal received by the ultrasonic probe 102 with a predetermined gain. The ultrasonic signal transmission and reception control unit 107 is for controlling the signal transmission unit 105 and the signal receiving unit 106. The phasing addition unit 108 controls the phase of the RF signal amplified by the signal receiving unit 106 to form ultrasonic beams corresponding to one or a plurality of convergent points, thereby generating RF signal frame data.

The RF signal frame data generated by the phasing addition unit 108 is stored in a data storage unit 109. A two-dimensional tomographic image forming unit 113 forms a two-dimensional tomographic image on the basis of the RF signal frame data stored in the data storage unit 109. The tomographic volume data generation unit 114 generates tomographic volume data by performing three-dimensional coordinate transformation of the two-dimensional tomographic image, which is formed by the two-dimensional tomographic image forming unit 113, on the basis of the acquisition position. A three-dimensional tomographic image forming unit 115 forms a three-dimensional tomographic image by performing volume rendering on the basis of the brightness and opacity of tomographic volume data.

A plurality of RF signal frame data items stored in the data storage unit 109 are appropriately output to a two-dimensional elasticity image forming unit 116, and a two-dimensional elasticity image is formed. The two-dimensional elasticity image formed by the two-dimensional elasticity image forming unit 116 is output to an elasticity volume data generation unit 117, and elasticity volume data is generated by performing three-dimensional coordinate transformation on the basis of the acquisition position of the two-dimensional elasticity image. A three-dimensional elasticity image forming unit 118 forms a three-dimensional elasticity image by performing volume rendering on the basis of the value of elasticity and opacity of elasticity volume data. A combination processing unit 119 is configured to combine a two-dimensional tomographic image and a two-dimensional elasticity image or combine a three-dimensional tomographic image and a three-dimensional elasticity image. A display unit 120 is configured to display a composite image combined by the combination processing unit 119 or an ultrasonic image of a two-dimensional tomographic image. In addition, the ultrasonic diagnostic apparatus 100 includes a control unit 103 that controls each of the components described above and an operating unit 104 for performing various inputs to the control unit 103, and the operating unit 104 includes a keyboard, a track ball, and the like.

Hereinafter, detailed configurations of the main units shown in Fig. 1 will be described. The two-dimensional tomographic image forming unit 113 acquires RF signal frame data output from the data storage unit 109 on the basis of the setting conditions in the control unit 103, and forms a two-dimensional tomographic image by performing signal processing, such as gain correction, log compression, detection, edge enhancement, and filtering, on the RF signal frame data. In this case, the ultrasonic probe 102 measures a signal transmission and reception direction ($\theta$, $\phi$) simultaneously with transmission and reception of an ultrasonic wave. Here, $\theta$ is a scan angle in the longitudinal direction, and $\phi$ is a scan (swing) angle in the short axis direction. The tomographic volume data generation unit 114 generates tomographic volume data by performing three-dimensional transformation of a plurality of two-dimensional tomographic images on the basis of the signal transmission and reception direction ($\theta$, $\phi$) equivalent to the acquisition position of the two-dimensional tomographic image.

The three-dimensional tomographic image forming unit 115 performs volume rendering using the following expressions (1) to (3) for forming a three-dimensional tomographic image from the tomographic volume data.

$$\mathrm{Cout(i) \ = \ Cout(i-1) \ + \ (1-Aout(i-1)) \cdot A(i) \cdot C(i) \cdot S(i) \ - \ (1)}$$

$$\mathrm{Aout(i) \ = \ Aout(i-1) \ + \ (1-Aout(i-1)) \cdot A(i) \qquad \quad - \ (2)}$$

$$\mathrm{A(i) \ = \ Opacity[C(i)] \qquad \qquad \qquad - \ (3)}$$

Here, C(i) is a brightness value of an i-th voxel on the line of sight when a three-dimensional tomographic image is viewed from a certain point on the created two-dimensional projection plane. Cout(i) is an output pixel value. For example, when the brightness values of N voxels are aligned on the line of sight, the brightness value Cout (N-1) obtained by integration from i = 0 to N-1 is a pixel value which is eventually output. Cout(i-1) indicates an integrated value up to the (i-1)-th value.

In addition, A(i) is the opacity of the brightness value of an i-th pixel on the line of sight, and is a tomographic opacity table that takes values of 0 to 1.0 as shown in expression (3). The tomographic opacity table determines the rate of contribution onto the two-dimensional projection plane (three-dimensional tomographic image) to output by referring to the opacity from the brightness value.

S(i) is a weight component for shading which is calculated from the brightness C(i) and the gradient calculated from the surrounding pixel values. For example, S(i) indicates the emphasis effect, such as "when a light source and the normal line of the plane having a voxel i at the center match each other, 1.0 is given since the strongest reflection occurs" and "when the light source and the normal line are perpendicular to each other, 0.0 is given".

Both Cout(i) and Aout(i) have 0 as their initial values. As shown in expression (2), Aout(i) is integrated whenever passing through a voxel and converges on 1.0. Accordingly, as shown in expression (1), when the integrated value Aout (i-1) of the opacity up to the (i-1)-th value reaches approximately 1.0, the brightness value C(i) from the i-th value is not reflected on the output image.

The two-dimensional elasticity image forming unit 116 measures a displacement from a plurality of RF signal frame data items stored in the data storage unit 109. The two-dimensional elasticity image forming unit 116 has an elasticity calculation section that calculates elasticity frame data indicating the elasticity distribution measured by scanning the object 101 in a three-dimensional manner using an ultrasonic wave. Then, the two-dimensional elasticity image forming unit 116 forms a two-dimensional elasticity image by calculating the value of elasticity on the basis of the measured displacement. The value of elasticity is any of the elasticity information including the strain, elastic modulus, displacement, viscosity, strain ratio, and the like. The elasticity volume data generation unit 117 generates elasticity volume data by performing three-dimensional transformation of a plurality of two-dimensional elasticity images on the basis of the signal transmission and reception direction $(\theta, \phi)$ equivalent to the acquisition position of the two-dimensional elasticity image. The three-dimensional elasticity image forming unit 118 forms a three-dimensional elasticity image by dividing the elasticity volume data into a plurality of items on the basis of the value of elasticity and performing volume rendering of the divided elasticity volume data items.

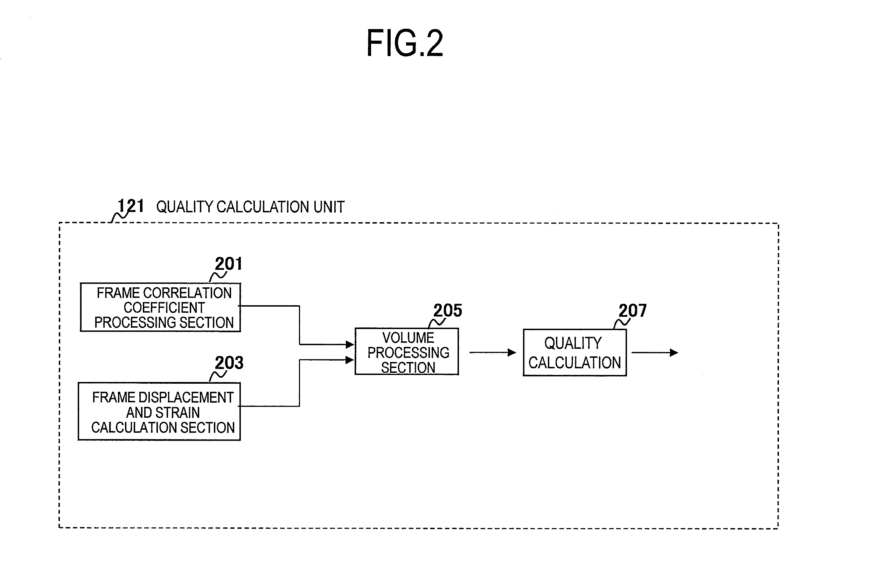

Hereinafter, a quality calculation unit 121 that is a characteristic unit of the present invention will be described. Fig. 2 is a block diagram showing the configuration of the quality calculation unit 121. Basically, the quality calculation unit 121 detects the quality, which is the quality of a three-dimensional elasticity image or information of compression, and calculates the quality of the elasticity volume data for the evaluation of good and bad. Here, as a quality to be detected, it is possible to use various qualities, such as autocorrelation, displacement, strain, and S/N ratio of strain of a pair of tomographic frame data items when calculating a two-dimensional elasticity image. In addition, it is also possible to use a correlation of elasticity frame data of the same position between a plurality of elasticity volume data items whose acquisition times are different.

The quality calculation unit 121 is configured to include a frame correlation processing section 201, a frame displacement and strain processing section 203, a volume processing section 205, and a quality calculation section 207. The frame correlation processing section 201 stores an autocorrelation between a pair of tomographic frame data items used when the two-dimensional elasticity image forming unit 116 calculates elasticity frame data, and calculates a frame evaluation value indicating the quality of the elasticity frame data of a frame unit.

In addition, the frame displacement and strain processing section 203 stores the pressure and the value of elasticity (strain, elastic modulus, displacement, viscosity, and strain ratio) calculated by the two-dimensional elasticity image forming unit 116 so as to match the elasticity frame data, and calculates a frame evaluation value indicating the quality of the elasticity frame data of a frame unit.

The volume processing section 205 calculates a volume evaluation value indicating the quality of the elasticity volume data by averaging the frame evaluation values calculated in units of frames by the frame correlation processing section 201 and frame displacement and strain processing section 203. Here, although one volume evaluation value is preferably calculated, a plurality of volume evaluation values may be calculated.

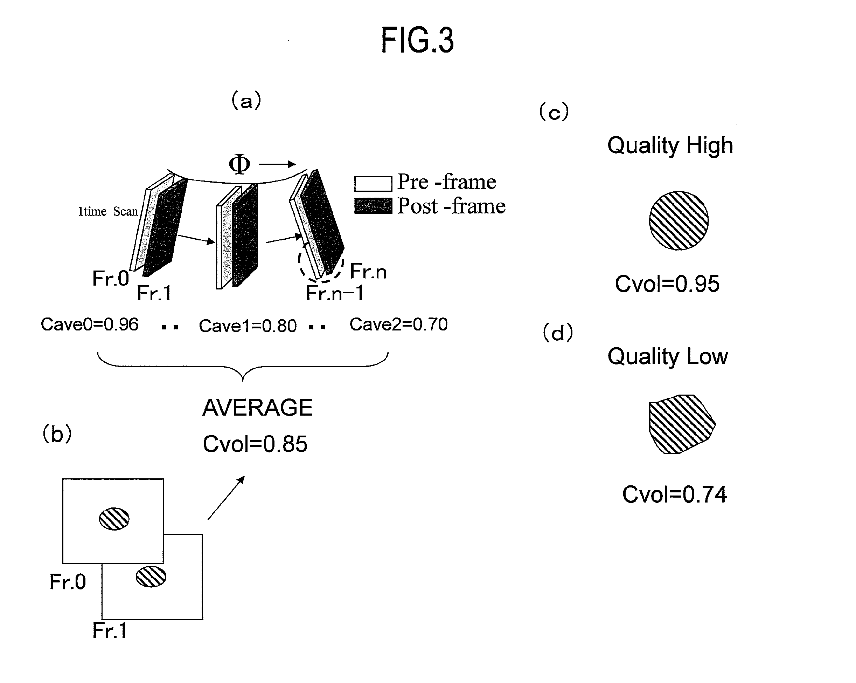

A detailed processing procedure of the first embodiment will be described with reference to Fig. 3. As shown in Fig. 3A, in a three-dimensional scan, an operation to measure tomographic frame data by performing an electronic scan in the longitudinal direction $(\theta)$ is performed while sliding the scanning cross-section in the short axis direction $(\phi)$, thereby obtaining tomographic frame data Fr.0 to Fr.n of a plurality of (n) frames. A mechanical scan or an electronic scan may be applied as the scan in the short axis direction $(\phi)$. For example, in the case of performing a scan by swinging the ultrasonic probe 102 using a motor, it is possible to control the motor speed to low speed or high speed.

In a three-dimensional scan, the two-dimensional elasticity image forming unit 116 estimates the displacement distribution of the body tissue by performing an autocorrelation operation between a pair of adjacent tomographic frame data items Fr.0 and Fr.1, for example. In addition, although not shown in the drawings, the displacement distribution of the body tissue may also be estimated by performing an autocorrelation operation between, for example, tomographic frame data Fr.0 acquired currently (real time) and tomographic frame data Fr.0 acquired in the past at the same position with reference to a plurality of tomographic volume data items of the same part acquired at different times which are stored in the tomographic volume data generation unit 114 shown in Fig. 1. The autocorrelation value calculated between a pair of tomographic frame data items as described above is a frame evaluation value indicating the quality of the elasticity frame data calculated by the pair of tomographic frame data items. In addition, "an autocorrelation value between a pair of tomographic frames is high" means that the degree of coincidence of the waveforms of a plurality of RF signals, which form tomographic frame data, is high and the quality of elasticity frame data obtained by these tends to be high.

Therefore, the frame correlation processing section 201 acquires an autocorrelation value between each pair of tomographic frames from the two-dimensional elasticity image forming unit 116, and calculates a frame average value Cave of the autocorrelation values in units of pixels for each item of elasticity frame data as shown in Figs. 3 (a) and 3 (b). Then, autocorrelation values corresponding to all elasticity frame data items that form the elasticity volume data are set as frame evaluation values and the average or the additional value is calculated, thereby calculating the volume evaluation value indicating the quality of the elasticity volume data. For example, if the average of the elasticity volumes of the autocorrelation values is Cvol = 0.95, "Quality High" is displayed on the display unit 120 as shown in Fig. 3(c) since the volume evaluation value indicating the quality is high. In addition, a three-dimensional elasticity image obtained by volume rendering of the elasticity volume data is not likely to break. On the other hand, as shown in Fig. 3(d), if the average of the autocorrelation values is Cvol = 0.74, "Quality Low" is displayed on the display unit 120. A three-dimensional elasticity image in this case has a large amount of noise. For example, an image, which is circular originally, is deformed and displayed, and becomes an image that lacks in the sharpness. In this manner, it is possible to evaluate the quality of the elasticity volume data by calculating the volume evaluation value indicating the quality for each item of elasticity volume data. In addition, a detailed embodiment of the frame displacement and strain calculation section 203 will be described later.

Second embodiment

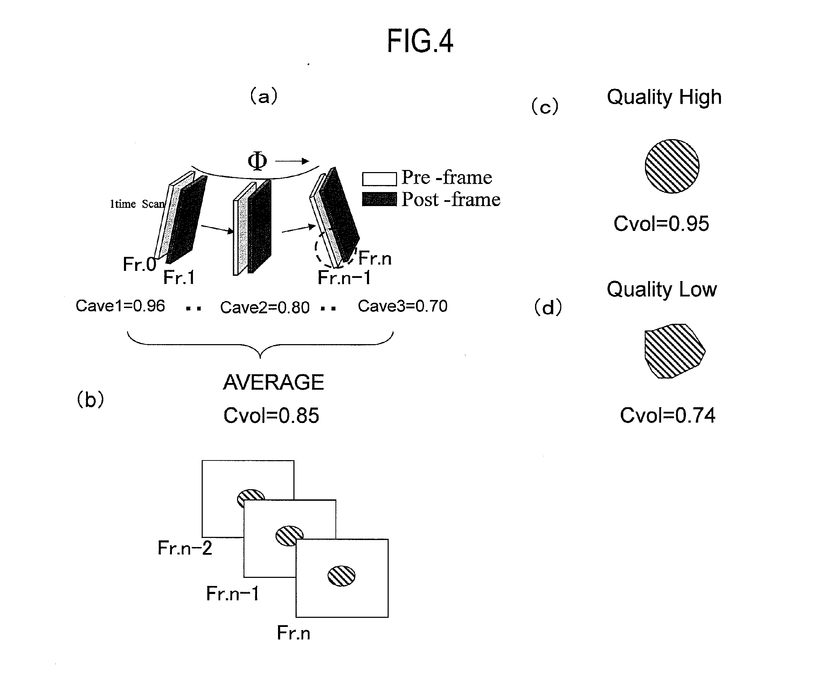

Referring to Fig. 4, a detailed processing procedure of the two-dimensional elasticity image forming unit 116 and the quality calculation unit 121 of a second embodiment of the present invention will be described. A difference between the first and second embodiments is the processing procedure of the two-dimensional elasticity image forming unit 116 and the frame correlation processing section 201. Since others are the same as those in the first embodiment, explanation thereof will be omitted. In the first embodiment, the two-dimensional elasticity image forming unit 116 estimates the displacement distribution of the body tissue by performing an autocorrelation operation between a pair of adjacent tomographic frame data items. In contrast, in the second embodiment, the two-dimensional elasticity image forming unit 116 estimates the displacement distribution of the body tissue by performing an autocorrelation operation between current (real time) tomographic frame data and tomographic frame data of past several frames. In particular, tomographic frame data having a highest value of autocorrelation with the past several frames is selected to estimate the displacement distribution of the body tissue, thereby creating elasticity frame data. That is, the autocorrelation value and the displacement distribution data for one frame are calculated by a plurality of three-dimensional scans. Subsequent processing is the same as that in the first embodiment, and evaluation results shown in Figs. 4(c) and 4(d) are displayed.

According to the second embodiment, elasticity frame data with high autocorrelation value can be selected by a plurality of three-dimensional scans. Therefore, since high-quality elasticity volume data can be generated eventually, it is possible to improve the quality of a three-dimensional elasticity image.

Third embodiment

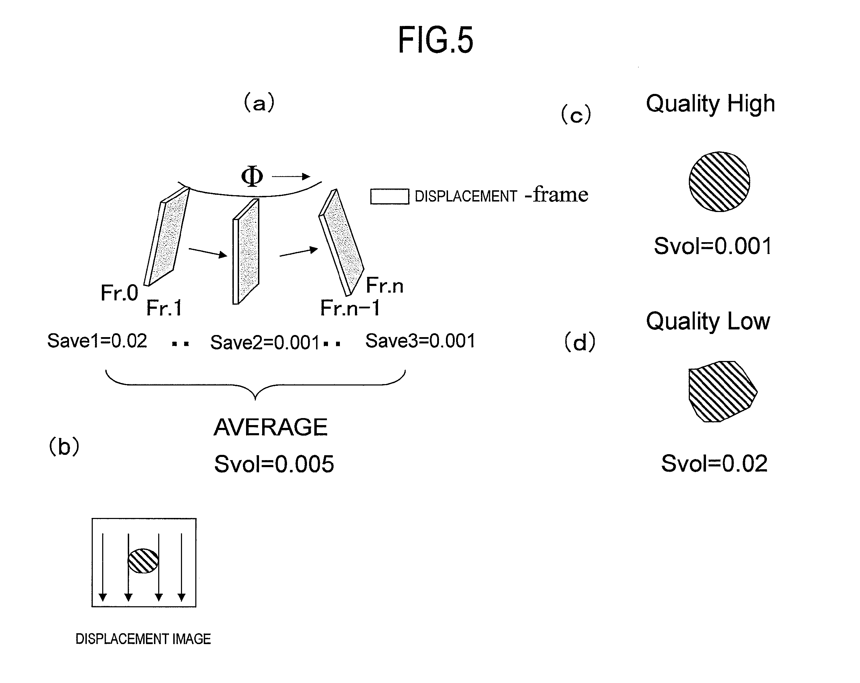

Referring to Fig. 5, a detailed processing procedure of the quality calculation unit 121 of a third embodiment of the present invention will be described. The third embodiment is different from the first and second embodiments in that the evaluation value of the quality of the elasticity frame data, which forms elasticity volume data, is calculated on the basis of at least one of the average of the value of elasticity (displacement, strain, strain ratio, viscosity, and elastic modulus) of elasticity frame data, an S/N ratio, and deviation information instead of the autocorrelation value of a pair of tomographic frame data items when calculating the elasticity frame data.

As shown in Fig. 5(a), elasticity (displacement) frame data detected from the autocorrelation between frames is acquired by a plurality of (n) frames by repeating a two-dimensional scan in the short axis direction. As a result, three-dimensional elasticity volume data is created as a group of two-dimensional elasticity frame data shown in Fig. 5(b). For example, an average of the displacement of the elasticity frame data is calculated, and this average is set as a volume evaluation value indicating the quality of the elasticity volume data. In addition, it is possible to detect a variation of an image by using the variance of the average of the displacement of each frame. The variance Save of the average of each frame is calculated by expression (4). In addition, an elasticity volume data average Svol of the variance Save of all elasticity frame data items, which form the elasticity volume data, is calculated by expression (5), and is set as a volume evaluation value indicating the quality of the elasticity volume data.

$$Save = \sum_{j=-W/2}^{+W/2} \sum_{i=-H/2}^{+H/2} (d(i,j) - d^*)^2 \qquad - (4)$$

Here, W is a horizontal width of an image, H is a height of an image, d(i, j) is a displacement, and d* is an average of the displacement.

$$Svol = \frac{\sum_{\phi=\phi_0}^{\phi N} Save(\phi)}{N_\phi} \qquad - (5)$$

According to the present embodiment, since noise decreases as a variation decreases, high evaluation is given in the case of a small value like Svol = 0.001 and a volume evaluation value "Quality High" is displayed, as shown in Fig. 5(c). This also becomes a toned image as a three-dimensional elasticity image. On the other hand, in a noisy image, the variation increases. Therefore, as shown in Fig. 5(d), low evaluation is given in the case of a large value like Svol = 0.02, and a volume evaluation value "Quality Low" is displayed. This image also becomes an image that lacks in the sharpness.

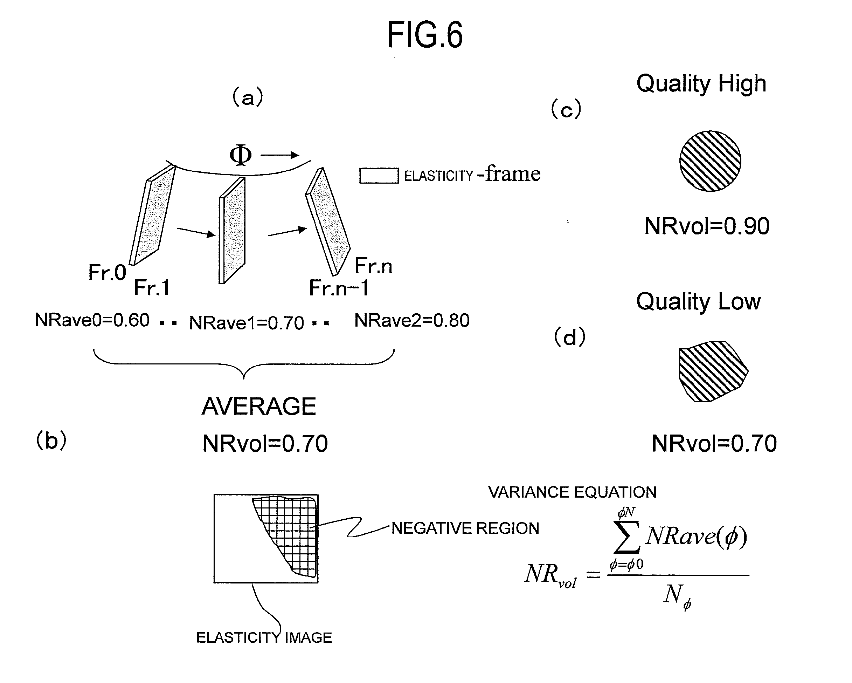

Fig. 6 is a modification of the present embodiment, and is an example where the quality of the elasticity volume data is evaluated on the basis of strain frame data instead of the displacement frame data. As shown in Fig. 6(a), elasticity volume data is formed by strain frame data of a plurality of (n) frames. Generally, if pressure is applied to the body tissue, there can be no negative strain. Accordingly, as shown in Fig. 6(b), if a negative strain is mixed in one strain frame data item, the average strain is reduced. Therefore, an average of the average strain of each frame is calculated for each item of the strain frame data in the φ direction, and this average is set as a volume evaluation value indicating the quality of the elasticity volume data. According to this, "Quality High" is displayed as shown in Fig. 6(c) in the case of high evaluation, and "Quality Low" is displayed as shown in Fig. 6(d) in the case of low evaluation.

Fourth embodiment

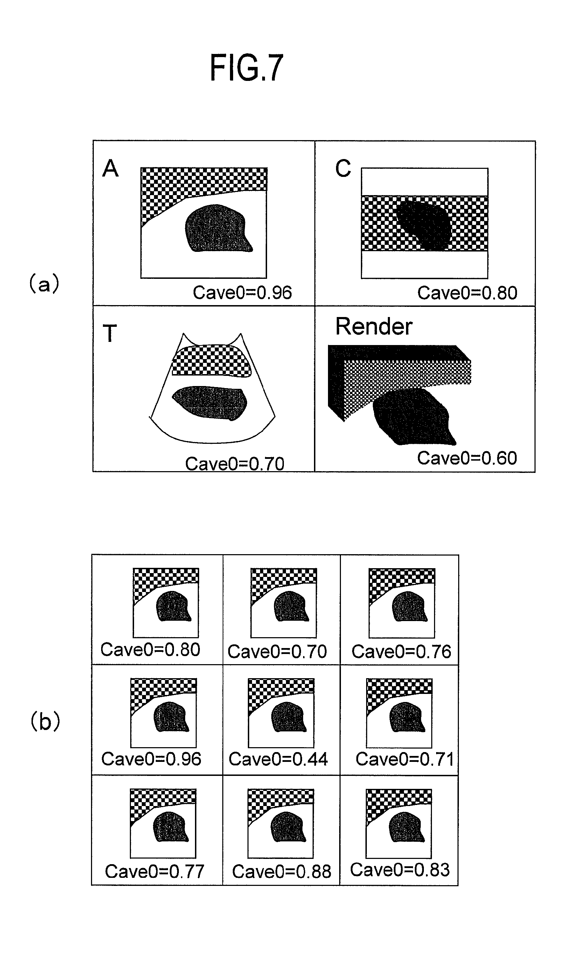

Fig. 7 shows examples of various kinds of display images that are formed using the elasticity volume data calculated according to each embodiment of the present invention. In the examples shown in Fig. 7, when displaying a cross-sectional image in a certain cross-section using the elasticity volume data, a volume evaluation value indicating the quality is displayed in each cross-sectional image displayed. Specifically, as shown in Fig. 7(a), a volume evaluation value indicating the quality is displayed in each of an axial plane A, a theta plane T, and a coronal plane C of three arbitrary orthogonal cross-sections and a three-dimensional elasticity image Render. In addition, as shown in Fig. 7(b), also in the case of a so-called multi-slice cross-sectional image, the volume evaluation value indicating the quality can be displayed in each display of a plurality of cross-sectional images.

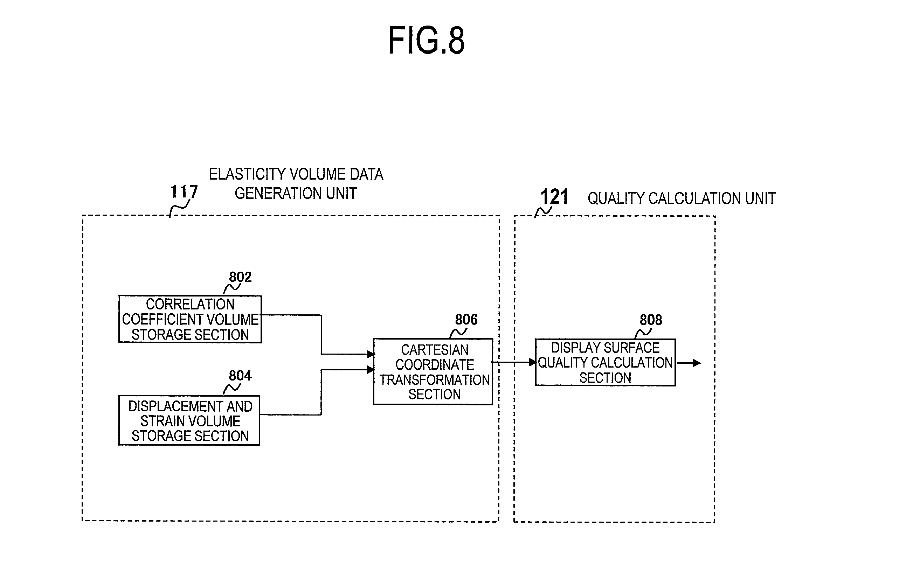

In order to realize the image display example shown in Fig. 7, the elasticity volume data generation unit 117 and the quality calculation unit 121 are configured as shown in Fig. 8. That is, in the elasticity volume generation unit 117, the autocorrelation value calculated by the two-dimensional elasticity image forming unit 116 is stored in an autocorrelation value volume storage section 802 or a displacement and strain volume storage section 804 of the elasticity volume generation unit 117. This data is data based on the dimension of RF array data curve coordinates (RTφ) of an ultrasonic wave, and is converted into data based on the Cartesian coordinates by a Cartesian coordinate transformation section 806. The control unit 103 causes a display surface quality calculation section 808 to calculate a volume evaluation value indicating the quality of each cross-section for the coordinate plane referred to for display.

Fifth embodiment

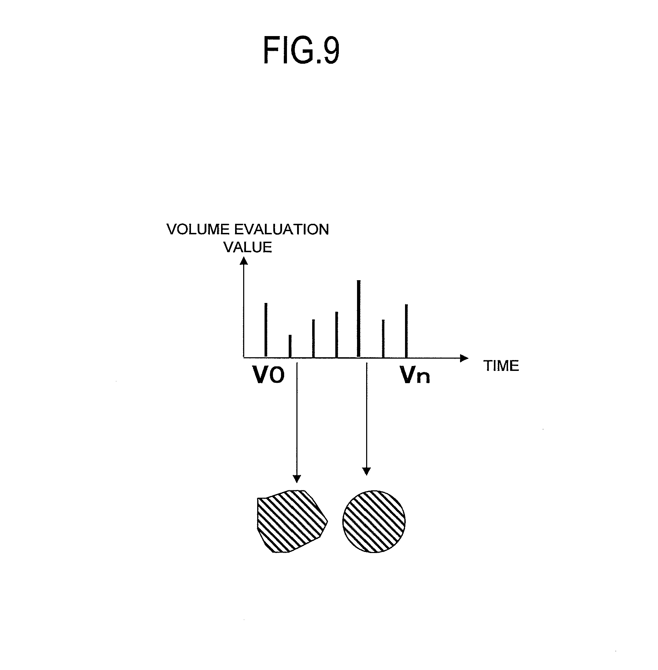

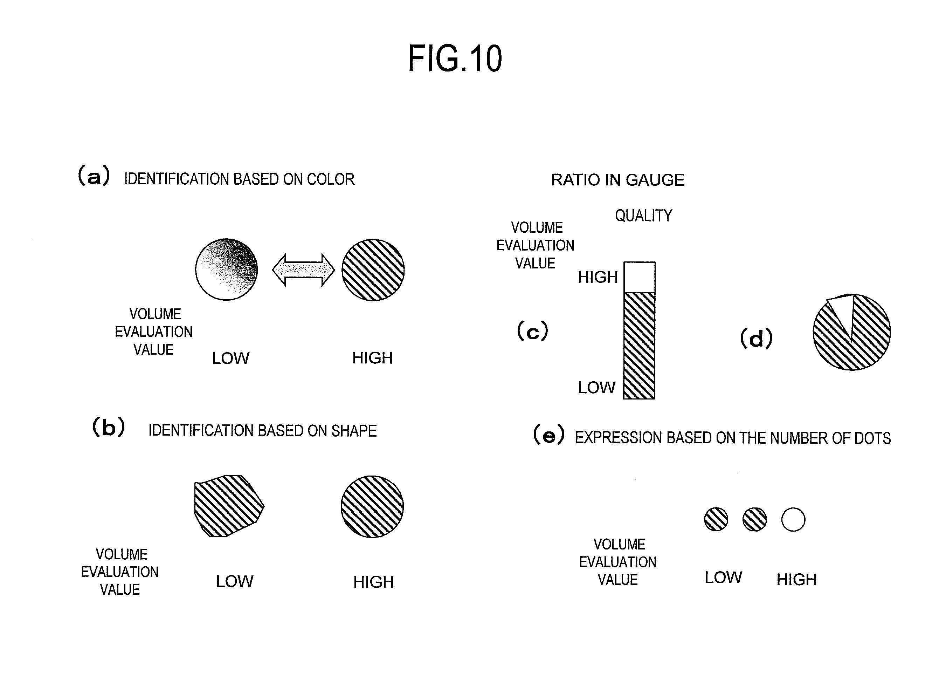

Referring to Figs. 9 and 10, an embodiment of a method of displaying the volume evaluation value indicating the elasticity volume data evaluated by each embodiment will be described. In Fig. 9, the horizontal axis indicates time, and the vertical axis indicates a volume evaluation value indicating the quality. Accordingly, volume evaluation values of a plurality of elasticity volume data items V0 to Vn acquired continuously can be seen at a glance. In addition, the same marks as in Fig. 3 are displayed so as to correspond to elasticity volume data with the highest volume evaluation value and elasticity volume data with the lowest volume evaluation value.

Fig. 10 shows a modification of marks, bar charts, pie charts, and others that express volume evaluation values indicating the quality of elasticity volume data or a three-dimensional elasticity image. Fig. 10(a) is an example where the display color of a circular mark is changed corresponding to the volume evaluation value so that the level of the volume evaluation value can be identified at a glance. Fig. 10 (b) is the same as the mark shown in the first embodiment and the like, and is an example where the level of the volume evaluation value is expressed according to the degree of circularity of the circular mark. Fig. 10(c) is an example where the level of the volume evaluation value is expressed according to the ratio in the gauge of a bar chart. Fig. 10 (d) is an example where the level of the volume evaluation value is expressed according to the ratio of a pie chart. Fig. 10 (e) is an example where a plurality of small circles are aligned and the ratio of the display forms of the small circles is changed according to the volume evaluation value.

Sixth embodiment

The display form and storage form of a three-dimensional elasticity image that is formed using the elasticity volume data calculated according to each embodiment of the present invention will be described.

The display unit 120 displays a three-dimensional elasticity image on the basis of the volume evaluation value indicating the quality of the elasticity volume data calculated by the quality calculation unit 121. When the volume evaluation value is higher than the reference value of display, the control unit 103 gives an instruction to display the three-dimensional elasticity image, and the display unit 120 displays the three-dimensional elasticity image whose volume evaluation value is higher than the reference value of display. When the volume evaluation value is lower than the reference value of display, the control unit 103 gives an instruction not to display the three-dimensional elasticity image, and the display unit 120 does not display the three-dimensional elasticity image whose volume evaluation value is lower than the reference value of display.

The reference value of display is set to 0.95, for example. In addition, the operator can set the reference value of display through the operating unit 104.

According to the present embodiment, when the volume evaluation value indicating the quality of elasticity volume data is higher than the reference value of display, the display unit 120 displays the three-dimensional elasticity image. That is, the display unit 120 can display only the three-dimensional elasticity image whose volume evaluation value indicating the quality of elasticity volume data is higher than the reference value of display.

In addition, a storage unit (not shown) stores the three-dimensional elasticity image on the basis of the volume evaluation value indicating the quality of the elasticity volume data calculated by the quality calculation unit 121. When the volume evaluation value is higher than the reference value of storage, the control unit 103 gives an instruction to store the three-dimensional elasticity image in the storage unit, and the storage unit stores the three-dimensional elasticity image whose volume evaluation value is higher than the reference value of storage. When the volume evaluation value is lower than the reference value of storage, the control unit 103 gives an instruction not to store the three-dimensional elasticity image in the storage unit, and the storage unit does not store the three-dimensional elasticity image whose volume evaluation value is lower than the reference value of storage.

According to the present embodiment, the storage unit that stores a three-dimensional elasticity image when the volume evaluation value indicating the quality of elasticity volume data is higher than the reference value of storage is provided. That is, the storage unit can store only the three-dimensional elasticity image whose volume evaluation value indicating the quality of elasticity volume data is higher than the reference value of storage.

The reference value of storage can be set similar to the reference value of display. The operator can set the reference value of storage through the operating unit 104. The reference value of display and the reference value of storage may be set to be the same.

As described above, according to the present invention, an ultrasonic diagnostic apparatus includes an elasticity volume generation unit 117 that generates elasticity volume data by collecting a plurality of elasticity frame data items indicating the elasticity distribution measured by scanning an object in a three-dimensional manner using an ultrasonic wave, a three-dimensional elasticity image forming unit 118 that forms a three-dimensional elasticity image by performing volume rendering of the elasticity volume data, and a display unit 120 that displays the three-dimensional elasticity image, and is characterized in that it includes a quality calculation unit 121 that calculates a volume evaluation value indicating the quality of the elasticity volume data on the basis of a frame evaluation value indicating the quality of the elasticity frame data. In addition, an evaluation calculation method includes a step of generating elasticity volume data by collecting a plurality of elasticity frame data items indicating an elasticity distribution measured by scanning an object in a three-dimensional manner using an ultrasonic wave; a step of forming a three-dimensional elasticity image by performing volume rendering of the elasticity volume data; a step of displaying the three-dimensional elasticity image; and a step of calculating a volume evaluation value indicating the quality of the elasticity volume data on the basis of a frame evaluation value indicating the quality of the elasticity frame data.

That is, the high frame evaluation value indicating the quality of elasticity frame data means that the elasticity frame data is calculated in a stable measurement state. In view of this, the present invention is configured to perform evaluation by calculating the frame evaluation value for evaluating the quality of elasticity frame data and calculating the volume evaluation value, which indicates the quality of elasticity volume data, on the basis of the frame evaluation value indicating a plurality of elasticity frame data items that form the elasticity volume data. Thus, since a method of evaluating the quality of elasticity volume data is established, the quality of a three-dimensional elasticity image can be easily improved by acquiring or selecting the elasticity volume data with a high volume evaluation value indicating the quality.

Here, the quality of elasticity frame data or the quality evaluation value means that the elasticity frame data is measured in an appropriate compression state that is stable. Similarly, the quality of elasticity volume data or the quality evaluation value means a group of elasticity frame data with high quality or high quality evaluation values. Accordingly, with the high quality or the high quality evaluation value, it is possible to generate a three-dimensional elasticity image with a small amount of noise as a result.

Incidentally, the frame evaluation value indicating the quality of elasticity frame data can be calculated by the quality evaluation method. For example, "an autocorrelation value between a pair of tomographic frame data items, which are a basis for calculating the elasticity frame data, is high" means that the degree of coincidence of a pair of tomographic frame data items is high and the pair of tomographic frame data items has been measured in a stable measurement state. Therefore, it is possible to perform evaluation by setting the autocorrelation value between the pair of tomographic frame data items, which are a basis for calculating the elasticity frame data, as a frame evaluation value and calculating the volume evaluation value indicating the quality of the elasticity volume data on the basis of the frame evaluation value. However, as will be described later, the frame evaluation value related to the present invention is not limited to the autocorrelation value between a pair of tomographic frame data items.

In the present invention, the quality calculation unit 121 may be configured to set an autocorrelation value between a pair of tomographic frame data items as a frame evaluation value indicating the quality of the elasticity frame data and calculate the volume evaluation value indicating the quality of the elasticity volume data on the basis of an additional value or an average value of the frame evaluation values of all items of the elasticity frame data that forms the elasticity volume data. In this case, as the autocorrelation value between a pair of tomographic frame data items, it is possible to use an autocorrelation value between a pair of tomographic frame data items temporally adjacent to each other in a three-dimensional scan. In addition, instead of this, as the autocorrelation value between a pair of tomographic frame data items, it is also possible to use an autocorrelation value between the pair of tomographic frame data items, which are a basis for calculating the elasticity frame data of the same scan plane position, among the plurality of elasticity frame data items that form each of a plurality of elasticity volume data items repeatedly generated by the elasticity volume generation unit.

In addition, as the autocorrelation value between a pair of tomographic frame data items, it is also possible to use an autocorrelation value between a pair of tomographic frame data items having a highest autocorrelation value among the autocorrelation values calculated between current tomographic frame data items and a plurality of past tomographic frame data items. According to this, since it is possible to select high-quality elasticity frame data to form elasticity volume data, it is possible to further increase the volume evaluation value. That is, the elasticity volume data is generated by collecting the elasticity frame data calculated on the basis of a pair of tomographic frame data items between which the autocorrelation value is highest. In addition, as described above, the present invention is not limited to calculating the frame evaluation value on the basis of the autocorrelation value between a pair of tomographic frame data items which are a basis for calculating the elasticity frame data. Instead of this, it is also possible to calculate the frame evaluation value indicating the quality of each item of elasticity frame data on the basis of the average, deviation, or S/N ratio of the distribution of the value of elasticity of the elasticity frame data. For example, evaluation can be performed in such a manner that the quality of elasticity frame data is high if the S/N ratio of the value of elasticity is large. In addition, any one of the displacement, strain, strain ratio, viscosity, and elastic modulus may be used as the value of elasticity.

It is preferable to display the volume evaluation value indicating the quality, which has been calculated as described above, on the display unit 120 so as to correspond to the three-dimensional elasticity image. In this manner, it is possible to determine how high the quality of the displayed three-dimensional elasticity image is. In addition, the volume evaluation value indicating the quality can be identified at a glance by displaying, on the display unit, marks, bar charts, or pie charts having different display forms corresponding to the volume evaluation value indicating the quality. This can contribute to the improvement in the diagnostic accuracy.

In addition, the present invention is not limited to the three-dimensional elasticity image, and may be configured to include a cross-sectional elasticity image forming section that displays three orthogonal cross-sectional elasticity images, which are formed on the basis of the elasticity volume data, or multiple cross-sectional elasticity images, which are obtained by slicing using a plurality of parallel cross-sections, on the display unit, or may be configured to include a display surface quality calculation section that calculates a volume evaluation value, which indicates the quality of each cross-sectional elasticity image, using the quality calculation unit and displays the volume evaluation value on the display unit.

As described above, according to the present invention, since the quality of a three-dimensional elasticity image can be displayed, the examiner can select a three-dimensional elasticity image easily. In addition, since it is also possible to reconstruct and display the high-quality three-dimensional elasticity image, high-level diagnostic support is possible.
Reference Signs List

| | |
|---|---|
| 100: | ultrasonic diagnostic apparatus |
| 102: | ultrasonic probe |
| 103: | control unit |
| 104: | operating unit |
| 105: | signal transmission unit |
| 106: | signal receiving unit |
| 107: | signal transmission and reception control unit |
| 108: | phasing addition unit |
| 109: | data storage unit |
| 113: | two-dimensional tomographic image forming unit |
| 114: | tomographic volume data generation unit |
| 115: | three-dimensional tomographic image forming unit |
| 116: | two-dimensional elasticity image forming unit |
| 117: | elasticity volume data generation unit |
| 118: | three-dimensional elasticity image forming unit |
| 119: | combination processing unit |
| 120: | display unit |
| 121: | quality calculation unit |

## Claims

1. An ultrasonic diagnostic apparatus including an elasticity volume generation unit that generates elasticity volume data by collecting a plurality of elasticity frame data items indicating an elasticity distribution measured by scanning an object in a three-dimensional manner using an ultrasonic wave, a three-dimensional elasticity image forming unit that forms a three-dimensional elasticity image by performing volume rendering of the elasticity volume data, and a display unit that displays the three-dimensional elasticity image, the apparatus comprising:

   a quality calculation unit that calculates a volume evaluation value indicating a quality of the elasticity volume data on the basis of a frame evaluation value indicating a quality of the elasticity frame data.

2. The ultrasonic diagnostic apparatus according to claim 1,
   wherein the quality calculation unit sets an autocorrelation value between a pair of tomographic frame data items, which are a basis for calculating the elasticity frame data, as the frame evaluation value indicating the quality of the elasticity frame data and calculates the volume evaluation value indicating the quality of the elasticity volume data on the basis of an additional value or an average value of the frame evaluation values of all items of the elasticity frame data that form the elasticity volume data.

3. The ultrasonic diagnostic apparatus according to claim 2,
   wherein the quality calculation unit uses, as the autocorrelation value between the pair of tomographic frame data items, an autocorrelation value between a pair of tomographic frame data items temporally adjacent to each other in the three-dimensional scan.

4. The ultrasonic diagnostic apparatus according to claim 2,
   wherein the quality calculation unit uses, as the autocorrelation value between the pair of tomographic frame data items, an autocorrelation value between the pair of tomographic frame data items, which are a basis for calculating the elasticity frame data of the same scan plane position, among the plurality of elasticity frame data items that form each of a plurality of elasticity volume data items repeatedly generated by the elasticity volume generation unit.

5. The ultrasonic diagnostic apparatus according to claim 2,

wherein the quality calculation unit uses, as the autocorrelation value between the pair of tomographic frame data items, an autocorrelation value between a pair of tomographic frame data items having a highest autocorrelation value among the autocorrelation values calculated between current tomographic frame data items and a plurality of past tomographic frame data items.

6. The ultrasonic diagnostic apparatus according to claim 5,
wherein the elasticity volume data is generated by collecting the elasticity frame data calculated on the basis of a pair of tomographic frame data items between which the autocorrelation value is highest.

7. The ultrasonic diagnostic apparatus according to claim 1,
wherein the quality calculation unit calculates a frame evaluation value indicating the quality of each item of the elasticity frame data on the basis of an average, a deviation, or an S/N ratio of a distribution of a value of elasticity of the elasticity frame data, and calculates the volume evaluation value indicating the quality of the elasticity volume data by averaging the calculated frame evaluation values in units of the elasticity volume data.

8. The ultrasonic diagnostic apparatus according to claim 7,
wherein the value of elasticity is one of displacement, strain, a strain ratio, viscosity, and an elastic modulus.

9. The ultrasonic diagnostic apparatus according to claim 1,
wherein the volume evaluation value indicating the quality is displayed on the display unit so as to correspond to the three-dimensional elasticity image.

10. The ultrasonic diagnostic apparatus according to claim 9,
wherein the volume evaluation value indicating the quality is displayed on the display unit using marks, bar charts, or pie charts having different display forms corresponding to the volume evaluation value.

11. The ultrasonic diagnostic apparatus according to claim 1,
wherein the elasticity volume data generation unit includes a cross-sectional elasticity image forming section that displays three orthogonal cross-sectional elasticity images, which are formed on the basis of the elasticity volume data, or multiple cross-sectional elasticity images, which are obtained by slicing using a plurality of parallel cross-sections, on the display unit, and
the quality calculation unit includes a display surface quality calculation section that calculates a volume evaluation value, which indicates a quality of each of the cross-sectional elasticity images, and displays the volume evaluation value on the display unit.

12. The ultrasonic diagnostic apparatus according to claim 1,
wherein, when the volume evaluation value indicating the quality of the elasticity volume data is higher than a reference value of display, the display unit displays the three-dimensional elasticity image.

13. The ultrasonic diagnostic apparatus according to claim 1, further comprising:

a storage unit that stores the three-dimensional elasticity image when the volume evaluation value indicating the quality of the elasticity volume data is higher than a reference value of storage.

14. An evaluation calculation method comprising:

a step of generating elasticity volume data by collecting a plurality of elasticity frame data items indicating an elasticity distribution measured by scanning an object in a three-dimensional manner using an ultrasonic wave;
a step of forming a three-dimensional elasticity image by performing volume rendering of the elasticity volume data;
a step of displaying the three-dimensional elasticity image; and
a step of calculating a volume evaluation value indicating a quality of the elasticity volume data on the basis of a frame evaluation value indicating a quality of the elasticity frame data.

FIG.1

# FIG.2

EP 2 612 599 A1

**121** QUALITY CALCULATION UNIT

**201**

FRAME CORRELATION
COEFFICIENT
PROCESSING SECTION

**203**

FRAME DISPLACEMENT
AND STRAIN
CALCULATION SECTION

**205**

VOLUME
PROCESSING
SECTION

**207**

QUALITY
CALCULATION

# FIG.3

（a）

$\Phi \rightarrow$

1time Scan

☐ Pre -frame
■ Post -frame

Fr.0
Fr.1

Fr.n
Fr.n−1

Cave0=0.96 ·· Cave1=0.80 ·· Cave2=0.70

AVERAGE
Cvol=0.85

（b）

Fr.0
Fr.1

（c）

Quality High

Cvol=0.95

（d）

Quality Low

Cvol=0.74

EP 2 612 599 A1

# FIG.4

(a)

Φ →

1time Scan

☐ Pre -frame
■ Post -frame

Fr.0
Fr.1

Fr.n
Fr.n−1

Cave1=0.96 ·· Cave2=0.80 ·· Cave3=0.70

AVERAGE

(b)
Cvol=0.85

Fr.n−2

Fr.n−1

Fr.n

(c) Quality High

Cvol=0.95

(d) Quality Low

Cvol=0.74

EP 2 612 599 A1

# FIG.5

(a)

(c) Quality High

Svol=0.001

$\Phi \longrightarrow$

☐ DISPLACEMENT -frame

Fr.0
Fr.1

Fr.n
Fr.n−1

Save1=0.02 ·· Save2=0.001·· Save3=0.001

(d) Quality Low

Svol=0.02

AVERAGE

(b)

Svol=0.005

DISPLACEMENT IMAGE

EP 2 612 599 A1

# FIG.6

(a)

Fr.0 Fr.1 Fr.n-1 Fr.n

$\Phi \longrightarrow$

☐ ELASTICITY -frame

NRave0=0.60 ·· NRave1=0.70 ·· NRave2=0.80

AVERAGE
NRvol=0.70

(b)

NEGATIVE REGION

ELASTICITY IMAGE

(c)

Quality High

NRvol=0.90

Quality Low

(d)

NRvol=0.70

VARIANCE EQUATION

$$NR_{vol} = \frac{\sum_{\phi=\phi 0}^{\phi N} NRave(\phi)}{N_{\phi}}$$

# FIG.7

(a)

| A | C |
|---|---|
| Cave0=0.96 | Cave0=0.80 |
| T | Render |
| Cave0=0.70 | Cave0=0.60 |

(b)

| | | |
|---|---|---|
| Cave0=0.80 | Cave0=0.70 | Cave0=0.76 |
| Cave0=0.96 | Cave0=0.44 | Cave0=0.71 |
| Cave0=0.77 | Cave0=0.88 | Cave0=0.83 |

# FIG.8

ELASTICITY VOLUME DATA
**117** GENERATION UNIT

**121** QUALITY CALCULATION UNIT

**802**
CORRELATION
COEFFICIENT VOLUME
STORAGE SECTION

**804**
DISPLACEMENT AND
STRAIN VOLUME
STORAGE SECTION

**806**
CARTESIAN
COORDINATE
TRANSFORMATION
SECTION

**808**
DISPLAY SURFACE
QUALITY CALCULATION
SECTION

EP 2 612 599 A1

## FIG.9

# FIG.10

**(a)** IDENTIFICATION BASED ON COLOR

VOLUME
EVALUATION
VALUE          LOW                HIGH

RATIO IN GAUGE

QUALITY

VOLUME
EVALUATION
VALUE
        HIGH

**(c)**                        **(d)**

        LOW

**(b)** IDENTIFICATION BASED ON SHAPE

VOLUME
EVALUATION
VALUE          LOW                HIGH

**(e)** EXPRESSION BASED ON THE NUMBER OF DOTS

VOLUME
EVALUATION
VALUE          LOW      HIGH

EP 2 612 599 A1

## INTERNATIONAL SEARCH REPORT

| | International application No. |
| --- | --- |
| | PCT/JP2011/066176 |

**A.  CLASSIFICATION OF SUBJECT MATTER**
*A61B8/08*(2006.01)i

According to International Patent Classification (IPC) or to both national classification and IPC

**B.   FIELDS SEARCHED**

Minimum documentation searched (classification system followed by classification symbols)
A61B8/08

Documentation searched other than minimum documentation to the extent that such documents are included in the fields searched
| | | |
| --- | --- | --- |
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
| --- | --- | --- |
| X | JP 2008-259555 A  (Hitachi Medical Corp.), | 1,7,8,14 |
| Y | 30 October 2008 (30.10.2008), paragraphs [0063] to [0067] (Family: none) | 2-6,9-13 |
| Y | WO 2007/083745 A1  (Hitachi Medical Corp.), 26 July 2007 (26.07.2007), paragraphs [0048] to [0057] & US 2010/0220901 A1      & EP 1980210 A1 & CN 101370431 A | 2-6,9-13 |
| Y | JP 2010-119630 A  (GE Medical Systems Global Technology Co., L.L.C.), 03 June 2010 (03.06.2010), paragraphs [0052] to [0061] (Family: none) | 3,5,6,12,13 |

| ☒  Further documents are listed in the continuation of Box C. | ☐   See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 04 August, 2011 (04.08.11) | 13 September, 2011 (13.09.11) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/066176

| C (Continuation).   DOCUMENTS CONSIDERED TO BE RELEVANT | | |
|---|---|---|
| Category* | Citation of document, with indication, where appropriate, of the relevant passages | Relevant to claim No. |
| Y | US 6558324 B1  (Siemens Medical Solutions, Inc., USA),<br>06 May 2003 (06.05.2003),<br>column 15, line 38 to column 16, line 59;<br>fig. 6<br>(Family: none) | 9-11 |
| A | WO 2008/010500 A1  (Hitachi Medical Corp.),<br>24 January 2008 (24.01.2008),<br>entire text; all drawings<br>& US 2009/0292205 A1 | 1-14 |
| A | JP 2010-12311 A  (Hitachi Medical Corp.),<br>21 January 2010 (21.01.2010),<br>entire text; all drawings<br>(Family: none) | 1-14 |
| A | JP 2010-82308 A  (GE Medical Systems Global Technology Co., L.L.C.),<br>15 April 2010 (15.04.2010),<br>entire text; all drawings<br>(Family: none) | 1-14 |

Form PCT/ISA/210 (continuation of second sheet) (July 2009)

**EP 2 612 599 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008259555 A